# EUROPÄISCHE PATENTSCHRIFT

(11) **EP 0 825 880 B1**
(45) Veröffentlichungstag und Bekanntmachung des Hinweises auf die Patenterteilung: **02.01.2004**
(21) Anmeldenummer: 96919781.3
(22) Anmeldetag: 15.05.1996
(51) Int. Cl.: A61M 1/36

(54) **VORRICHTUNG ZUR BESTRAHLUNG VON KÖRPERFLÜSSIGKEITEN MIT UV-LICHT**
DEVICE FOR THE IRRADIATION OF BODILY FLUIDS WITH UV LIGHT
DISPOSITIF PERMETTANT D'EXPOSER DES LIQUIDES ORGANIQUES A DE LA LUMIERE ULTRAVIOLETTE

(30) Priorität: 17.05.1995 DE 19518117; 29.08.1995 DE 19531751
(43) Veröffentlichungstag der Anmeldung: 04.03.1998
(73) Patentinhaber: Müller, Hans, 81547 München (DE)
(72) Erfinder: Müller, Hans, 81547 München (DE)
(74) Vertreter: Fleuchaus, Leo, Dipl.-Ing.
(86) Internationale Anmeldenummer: PCT/EP1996/002115
(87) Internationale Veröffentlichungsnummer: WO 1996/036375

(56) Entgegenhaltungen:
- CH-A- 324 906
- DE-A- 2 453 556
- GB-A- 1 146 162
- GB-A- 2 053 445
- GB-A- 2 200 020
- US-A- 2 309 124

## Beschreibung

Die Erfindung betrifft eine Vorrichtung zur Bestrahlung von Körperflüssigkeiten, insbesondere Blut und/oder Gewebsextrakten, mit UV-Licht nach den Oberbegriffen des unabhängigen Anspruchs 1, sowie ein in einer solchen Vorrichtung verwendetes Aufnahmegefäß nach dem Oberbegriff des Anspruch 2 für die zu bestrahlenden Körperflüssigkeitsproben.

Derartige Vorrichtungen und Aufnahmegefäße sind z.B. insbesondere aus der US-A-2 309 124 bekannt und dienen bei einer Reihe bekannter Therapieverfahren dazu, einem Patienten Körperflüssigkeiten, wie z. B. Blut und/oder Gewebsextrakte zu entnehmen, um sie anschließend (neben gegebenenfalls weiteren Therapieschritten wie z. B. einer Sauerstoffanreicherung) einer UV-Bestrahlung zu unterziehen, bevor sie dem Patienten wieder appliziert werden.

Dabei ist es wünschenswert, eine gleichmäßige und vollständige Durchdringung des gesamten Stroms der bestrahlten Körperflüssigkeitsprobe mit UV-Strahlung zu erzielen.

Der Verwirklichung dieses Ziels stehen jedoch folgende Probleme im Weg: Zum ersten zeigt UV-Strahlung nur eine relativ geringe Eindringtiefe in Körperflüssigkeiten, insbesondere Blut. Weiterhin bilden sich aufgrund der relativ hohen Viskosität von Körperflüssigkeiten einerseits und der bei den angewandten Therapieverfahren andererseits auftretenden relativ niedrigen Strömungsgeschwindigkeiten in der Quarzglasküvette bevorzugt laminare Strömungen aus, welche ein Verwirbeln einzelner Flüssigkeitsschichten und damit eine gleichmäßige Vermischung des gesamten in der Quarzglasküvette fließenden Volumenstroms verhindern.

Im Ergebnis führt dies dazu, daß sich eine im wesentlichen stationäre Anordnung einzelner laminar übereinander abgleitender Flüssigkeitsschichten ausbildet, wobei die äußerste, also die unmittelbar mit der Innenwand der Quarzglasküvette in Berührung kommende Grenzschicht einen wesentlichen Teil der UV-Strahlung absorbiert, bevor diese radial weiter innenliegende Bereiche erreichen kann. Besonders gravierend ist dieses Problem dann, wenn die UV-Strahlung nur von einer Seite her auf die stationäre Küvette auftrifft, da dann nur der der UV-Lampe unmittelbar benachbart liegende Bereich der Küvette effektiv bestrahlt wird.

Bei der US-A-2 309 124 wird ein im wesentlichen flaches Aufnahmegefäß mit kreisrundem Querschnitt verwendet, durch den zu bestrahlendes Blut auf antiparallel zueinander geführten Teilströmungsabschnitten geführt wird. Die Teilströmungsabschnitte werden dabei durch quer angeordnete, an den jeweiligen Endabschnitten in Nähe der Kreisumfangswand durchbrochene Zwischenwänden definiert, wodurch sich eine mäanderförmig verschlungene Gesamtanordnung von Strömungsabschnitten ergibt. Da dabei das Blut an sich in jedem Teilströmungsabschnitt nur längs einer sich geradlinig erstreckenden Bahn geführt würde und lediglich beim Übergang von einem Teilströmungsabschnitt zum nächsten eine lokale Richtungsumlenkung und damit Verwirbelung des zu bestrahlenden Blutes erzielbar wäre, ist in der US-A-2 309 124 als zusätzliche Maßnahme vorgesehen, eine Anordnung paralleler, jeweils in sich verdrillter Querstäbe zwischen die Zwischenwände einzulegen, so daß in jedem Teilströmungsabschnitt der Blutstrom längs des verdrillten Querstabes geführt wird, so daß sich zusätzliche Zirkulationen im Blutstrom ausbilden können.

Ein in der DE-OS 19508279 offenbartes Bestrahlungsgerät ist mit einer in einem Gehäuse eingebauten UV-Lampe sowie einem in Form einer Quarzglasküvette ausgebildeten Aufnahmegefäß versehen, in welches die Körperflüssigkeit einzufüllen ist. Bei diesem Gerät wird durch Drehen der sich im Strahlungsbereich der UV-Lampe befindlichen Quarzglasküvette bezweckt, daß nicht nur eine kleine, unmittelbar mit Strahlung beaufschlagte Teilmenge der in der Küvette eingeschlossenen Körperflüssigkeit sehr intensiv bestrahlt wird, wie dies bei einer im Strahlungsbereich einer UV-Lampe ruhenden Quarzglasküvette der Fall wäre, sondern daß durch die Drehbewegung eine gleichmäßigere Bestrahlung der gesamten Körperflüssigkeitsprobe bewirkt wird.

Die durch die Drehbewegung der Küvette ausgelöste Zirkulation in der sich im Küvetteninneren befindlichen Flüssigkeit erzeugt aber in erster Linie ein laminares Aufeinanderabgleiten koaxial zueinander ausgerichteter hohlzylinderförmiger Flüssigkeitsschichten in Umfangsrichtung der zylinderförmigen Küvette. Dabei werden also vor allem wieder die in radialer Richtung außen gelegenen Randschichten in der Flüssigkeit bevorzugt bestrahlt, nicht jedoch die radial innenliegenden Bereiche.

Ziel der vorliegende Erfindung ist es deshalb Vorrichtungen und Aufnahmegefäße zur Bestrahlung von Körperflüssigkeiten mit UV-Licht bereitzustellen, bei welchen eine möglichst vollständige Verwirbelung und damit einhergehend gleichmäßige Bestrahlung des gesamten Volumens der Körperflüssigkeitsprobe erzielt wird, wobei aus Kostengründen sowie um eine möglichst universelle Verwendbarkeit in möglichst vielen Bestrahlungsvorrichtungen zu gewährleisten eine Lösung gesucht ist, die einen möglichst geringen apparativen Aufwand erfordert.

Diese Aufgabe wird durch Vorrichtungen zur Bestrahlung von Körperflüssigkeiten nach Anspruch 1 sowie durch ein Aufnahmegefäß nach Anspruch 2 gelöst. Die abhängigen Ansprüche 3 und 4 betreffen vorteilhafte Weitergestaltungen des erfindungsgemäßen Aufnahmegefäßes.

Die erfindungsgemäßen Vorrichtungen erlauben es, in einer einem Patienten entnommenen Körperflüssigkeitsprobe, welche durch ein Aufnahmegefäß hindurchströmt oder in einem sich drehenden Aufnahmegefäß eingeschlossen ist, eine ausreichende Verwirbelung der einzelnen Flüssigkeitsschichten zu erreichen, so daß im Laufe eines Bestrahlungsintervalls das gesamte Flüssigkeitsvolumen möglichst gleichmäßig mit den Innenwänden des Aufnahmegefässes in Berührung kommt, wo die höchste Intensität der einfallenden UV-Strahlung vorliegt.

Die Vorteile und Merkmale der vorliegenden Erfindung ergeben sich auch aus der nachfolgenden Beschreibung von Ausführungsbeispielen in Verbindung mit dem Anspruch und den Figuren.

Es zeigen:
- Fig. 1: einen Längschnitt durch eine erste erfindungsgemäße Bestrahlungsvorrichtung, bei der eine Körperflüssigkeit durch eine mit UV-Strahlung beaufschlagte Aufnahmevorrichtung strömt, in deren Innerem eine Schikanenvorrichtung für eine gleichmäßige Verwirbelung der Strömung sorgt;
- Fig. 2: einen Längschnitt durch eine zweite erfindungsgemäße Bestrahlungsvorrichtung, bei der eine Körperflüssigkeit in einer sich im Strahlungsbereich einer UV-Lampe drehenden Aufnahmevorrichtung eingeschlossen ist, wobei eine Schikanenvorrichtung im Innerem der Aufnahmevorrichtung für eine gleichmäßige Verwirbelung der zirkulierenden Strömung sorgt; und
- Fig. 3 und 4: perspektivische Darstellungen zweier Ausführungsformen einer in einer erfindungsgemäßen Bestrahlungsvorrichtung einzusetzenden Schikanenvorrichtung.

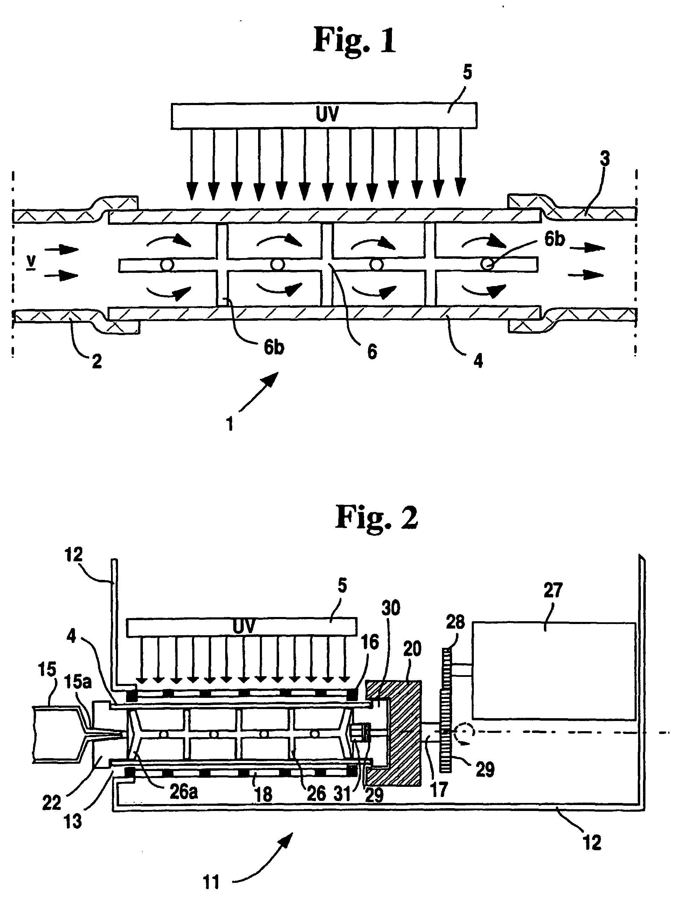

Fig. 1 zeigt eine Seitenansicht einer ersten erfindungsgemäßen Bestrahlungsvorrichtung 1, bei der eine Körperflüssigkeit wie z. B. Blut durch eine Zuleitung 2 sowie eine Ableitung 3 geführt in eine mit UV-Strahlung beaufschlagte Aufnahmevorrichtung 4 strömt, die in Form einer für UV-Strahlung durchlässigen Quarzglasküvette ausgeführt ist. Der lokale Strömungsvektor y in der strömenden Flüssigkeit ist dabei durch Pfeile angedeutet.

Aufgrund der bei bekannten medizinischen Therapieverfahren einzuhaltenden relativ niedrigen Strömungsgeschwindigkeiten von typischerweise maximal nur wenigen Zentimetern pro Minute käme es in der zylinderförmigen Küvette 4 ohne die Verwendung von Vorrichtungen, die eine Durchmischung der Flüssigkeit erzwingen, aufgrund der relativ hohen Viskosität von Blut zur Ausbildung der bekannten laminaren Rohrströmung mit parabelförmigen Geschwindigkeitsprofil, bei der in axialer Richtung einzelne hohlzylinderförmige Flüssigkeitsschichten aufeinander abgleiten, in radialer Richtung aber keinerlei Strömungskomponente auftritt.

In der in Fig. 1 gezeigten Anordnung würde dann aufgrund des hohen Absorptionsvermögens von Blut für UV-Strahlung nur die an der der UV-Lampe 5 zugewandten Innenwand der Küvette 4 gelegene Flüssigkeitsschicht intensiv bestrahlt, wohingegen in radialer Richtung weiter entfernte Flüssigkeitsschichten nicht oder kaum mehr bestrahlt würden.

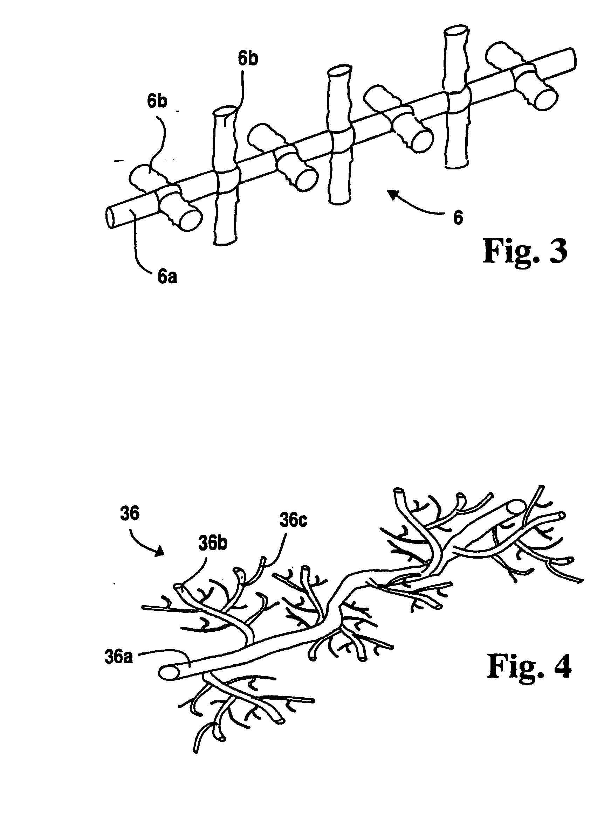

Dies wird durch Anbringen einer Schikanenvorrichtung 6 im Inneren der Küvette verhindert. Die Schikanenvorrichtung kann z.B. in Form eines in Fig.3 in perspektivischer Darstellung gezeigten "Verwirbelungsgestänges" ausgeführt sein, an dem an einer sich in Richtung der Küvettenachse verlaufenden Längsstange 6a eine Vielzahl von Querstangen 6b angebracht ist, wobei aufeinanderfolgende Querstangen um jeweils 90° zueinandender gedreht angeordnet sind. Durch diese Querstangen 6b wird eine laminare Rohrströmung im Inneren der Küvette weitestgehend verhindert, so daß im Küvetteninneren Strömungsvektoren v auftreten, die Komponenten in radialer Richtung aufweisen, wodurch es zu der gewünschten Verwirbelung in der strömenden Flüssigkeit kommt.

In Fig. 2 ist das Gehäuse 12 einer zweiten Ausführungsform einer erfindungsgemäßen Bestrahlungsvorrichtung 11 im Teilschnitt zu sehen. Das Gehäuse 12 weist eine Aufnahmeöffnung 13 zum Einführen einer Küvette 4 auf. Die Aufnahmeöffnung 13 führt in das Innere eines Führungsrohrs 16, welches zu einer Antriebswelle 17 axial ausgerichtet ist und z. B. an der Gehäuseinnenseite im Bereich der Aufnahmeöffnung 13 befestigt ist.

Das Führungsrohr 16 besteht z. B. aus einem für UV-Strahlung einer im Gehäuse angebrachten UV-Strahlungsquelle 5 durchlässigen Material oder, wie in Fig. 2 dargestellt, aus einem mit Schlitzen 18 zum Lichtdurchtritt versehenen Rohr.

Im Bereich des der Aufnahmeöffnung 13 im Gehäuse 12 entgegengesetzten Endes des Führungsrohrs 16 ist ein Adapter 20 vorgesehen, der auf einer Antriebswelle 17 aufsitzt, welche mittels eines aus zwei in einandergreifenden Zahnrädern 28, 29 bestehenden Untersetzungsgetriebes von einem Antriebsmotor 27 gedreht wird. Das in Einführungsrichtung vorne liegende Küvettenende ist in den Adapter 20 einschnappbar, und die Drehbewegung des Antriebsmotors 27 ist somit auf die Küvette 4 übertragbar.

Eine Küvette 4 kann mit einer Spritze 15, die z. B. eine einem Patienten entnommene Blut- oder Gewebsextraktsprobe enthält, durch Ansetzen des Spritzenkonus 15a an eine mittige Bohrung in einem Stopfen 22 befüllt werden. Am gegenüberliegenden Ende der Küvette 4 ist ein zweiter Stopfen 30 vorgesehen, der ebenfalls mit einer mittigen Bohrung versehen ist. In dieser zweiten Bohrung ist ein Bakterienfilter 29 mittels eines kleineren, ebenfalls durchbohrten Hilfsstopfens 31 gesichert. Beim Einfüllen der Körperflüssigkeit in die Küvette 4 kommt es dabei zu einem zum Druckausgleich benötigten Ausströmen von Luft aus der Küvette.

Die mit Körperflüssigkeit befüllte Küvette 4 wird samt der daran hängenden Spritze 15 durch die Aufnahmeöffnung 13 des Gehäuses 12 in das Bestrahlungsgerät 11 eingeschoben und in den Adapter 20 eingerastet. Beim Drehen des Adapters 20 mittels des Antriebsmotors 27 wird die Küvette samt Inhalt mitgedreht, wobei bei Wahl geeigneter Umdrehungsgeschwindigkeiten der gesamte Außenmantel der zylinderförmigen Küvette gleichmäßig in den Bereich der von der UV-Lampe 25 einfallenden Strahlung gebracht wird und sich im Inneren der Küvette eine Zirkularströmung in Umfangsrichtung einstellt.

Die Verwendung einer Schikanenvorrichtung 26 im Inneren der Küvette hat nun wiederum den Vorteil, daß es zu Verwirbelungen in der Zirkularströmung kommt, so daß Radialkomponenten in der Strömung auftreten, die für einen Flüssigkeitstransport zu den Innenwänden der Küvette und damit für eine möglichst homogenen Bestrahlung des gesamten Flüssigkeitsvolumens sorgen.

Nach Beendigung des Bestrahlungsvorgangs wird die Küvette 14 aus dem Adapter 20 ausgeklinkt und aus dem Gehäuse 12 entnommen. Anschließend kann z. B. der bestrahlte Inhalt mit der Spritze 15 wiederum aus der Küvette abgesaugt werden um dem Patienten zurückzuappliziert zu werden. Das Bakterienfilter 29 verhindert dabei eine Verkeimung des Küvetteninhalts beim Zurückströmen von Luft in die sich leerende Küvette.

Die in Fig. 3 gezeigte Schikanenvorrichtung 6 in Form eines "Verwirbelungsgestänges" hat den Vorteil, daß sie in kostengünstiger Weise als Kunststoffspritzgußteil hergestellt werden kann, und somit als steriler Einmalartikel nach erfolgter Bestrahlung einer Körperflüssigkeitsprobe in einer Quarzglasküvette zusammen mit derselben entsorgt werden kann.

Grundsätzlich können neben der in Fig. 3 gezeigten Ausführungsform jedoch auch beliebig anders geformte Schikanenvorrichtungen benutzt werden, solange dadurch eine ausreichende Verwirbelung im Inneren der Küvette erzielt wird.

Wird eine Schikanenvorrichtung in der in Fig. 2 gezeigten Küvette 4 verwendet, welche an ihren beiden Enden mit Stopfen 22, 30 verschlossen ist, so ist es besonders vorteilhaft, wenn an den beiden Enden der Längsstange der Schikanenvorrichtung ein schräg nach außen aufstehender Kranz von Lamellen 26a ausgebildet ist, welche dazu dienen, daß die in der Mitte gelegene Längsstange einen gewissen Abstand von den Bohrungen in der Mitte der die Küvette verschließenden Stopfen hält, so daß diese nicht blockiert werden und ein müheloses Befüllen und Entleeren der Küvette möglich ist.

Weiterhin kann die Schikanenvorrichtung z.B. statt in Form eines in Fig. 1 - 3 gezeigten "Verwirbelungsgestänges" auch in Form eines in Fig. 4 gezeigten "Korallenbäumchens" 36 ausgeführt sein, bei dem an einem sich im wesentlichen in Richtung der Küvettenachse verlaufenden Stamm 36a eine Vielzahl von unregelmäßig angeordneten Ästen 36b mit immer feiner werdenden, unregelmäßigen Verästelungen 36c angebracht ist. Durch diese feine Verästelungen 36c wird eine laminare Rohrströmung im Inneren der Küvette in besonders effektiver Weise verhindert, da die laminare Strömung immer wieder aufgerissen wird und somit eine weitgehende Verwirbelung der strömenden Flüssigkeit eintritt.

Im Gegensatz zu anderen denkbaren Mitteln um eine Verwirbelung im Inneren der Küvette zu erzielen, wie insbesondere Magnetrührvorrichtungen wie sie aus der chemischen Laborpraxis zur Vermischung von Flüssigkeiten oder zur Beschleunigung von Auflösungsvorgängen in Form von sich in einem äußeren zirkulierenden Magnetfeld rotierenden Magnetrührstäbchen bekannt sind, hat die Verwendung einer Schikanenvorrichtung im Inneren der zu bestrahlenden Küvette den Vorteil, daß sie wesentlich kostengünstiger ist und keinen weiteren apparativen Aufwand erfordert, wie er insbesondere zur Erzeugung derartiger Drehfelder notwendig wäre.

## Patentansprüche

1. Vorrichtung zur Bestrahlung von Körperflüssigkeitsproben, insbesondere Blut und/oder Gewebsextrakten mit UV-Licht, mit einem UV-Bestrahlungsgerät, welches eine UV-Lampe umfaßt, und mit einem Aufnahmegefäß für die zu bestrahlenden Körperflüssigkeitsproben, welches in den Strahlungsbereich der UV-Lampe einbringbar ist, wobei im Inneren dieses Aufnahmegefässes (4) eine Schikanenvorrichtung (6, 26, 36) zum Verwirbeln einer das Aufnahmegefäß (4) durchströmenden bzw. in dieser zirkulierenden Körperflüssigkeit vorgesehen ist, welche eine Vielzahl von der Körperflüssigkeit zu umströmende Schikanen (6b, 36b, 36c) umfaßt, wodurch eine laminare Strömung in der Körperflüssigkeit weitestgehend unterbunden wird,
**dadurch gekennzeichnet,**
**daß** das Aufnahmegefäß rohrartig ausgebildet und in eine Drehhalterung des UV-Bestrahlungsgerätes einsetzbar ist,
und **daß** die Schikanenvorrichtung in Form eines Verwirbelungsgestänges (6) mit einer in Richtung der Rohrachse verlaufenden Längsstange (6a) sowie davon abzweigenden Querstangen (6b) ausgeführt ist.

2. Aufnahmegefäß (4), welches durchlässig für UV-Strahlung ist und in dessen Innerem eine Schikanenvorrichtung (6, 26, 36) zum Verwirbeln einer das Aufnahmegefäß (4) durchströmenden bzw. in dieser zirkulierenden Körperflüssigkeit vorgesehen ist, welche eine Vielzahl von der Körperflüssigkeit zu umströmende Schikanen (6b, 36b, 36c) umfaßt, wodurch eine laminare Strömung in der Körperflüssigkeit weitestgehend unterbunden wird,
**dadurch gekennzeichnet,**
**daß** das Aufnahmegefäß rohrartig ausgebildet ist, daß sich die Schikanenvorrichtung über die Länge des Aufnahmegefäßes erstreckt und aus einer in Richtung der Rohrachse verlaufenden Längsstange (6a;36a) mit davon abzweigenden Querstangen (6b) oder mit einem im wesentlichen in Richtung der Rohrachse verlaufenden Stamm mit einer daran angebrachten Vielzahl von Ästen (36b) mit immer feiner werdenden, unregelmäßigen Verästelungen ausgeführt ist.

3. Aufnahmegefäß nach Anspruch 2, **dadurch gekennzeichnet,**
**daß** die von der Längsstange (6a) abzweigenden Querstangen (6b) um jeweils 90° gegeneinander versetzt sind.

4. Aufnahmegefäß nach Anspruch 2 oder 3, **dadurch gekennzeichnet,**
**daß** die Längstange (6a) an ihren beiden äußeren Enden mit einem Kranz schräg nach außen auftstehender Lamellen (26a) versehen ist.

## Claims

1. A device for irradiating body fluid samples, more specifically blood and/or tissue extracts, with ultraviolet light, said device having an ultraviolet irradiation apparatus comprising an ultraviolet light source and a reception container for receiving the body fluid samples to be irradiated, said reception container being movable into the radiation range of the ultraviolet light source, a baffle device (6, 26, 36), which is comprised of a plurality of baffle plates (6b, 36b, 36c) around which the body fluid will have to flow, being provided within said reception container (4) for turbulating a body fluid flowing through, or circulating in, said reception container (4), a laminar flow within the body fluid being largely prevented as a result thereof,
**characterized in that**
the reception container is configured to be tubular and is mountable in a rotating holder of the ultraviolet irradiation apparatus,
and that the baffle device is implemented in the form of a turbulence creating rod system (6) having a longitudinal rod (6a) oriented in the direction of the tube's axis and transverse rods (6b) branching off therefrom.

2. A reception container (4) that is translucent to ultraviolet light and within which there is provided a baffle device (6, 26, 36), which is comprised of a plurality of baffle plates (6b, 36b, 36c) around which the body fluid will have to flow, for turbulating a body fluid flowing through, or circulating in, said reception container (4), a laminar flow within the body fluid being largely prevented as a result thereof,
**characterized in that**
the reception container is configured to be tubular, that the baffle device extends along the length of the reception container and is implemented so as to consist of a longitudinal rod (6a; 36a) that is oriented in the direction of the tube's axis and has transverse rods (6b) branching off therefrom or of a trunk, which is substantially oriented in the direction of the tube's axis and has a plurality of branches (36b) ramifying into ever finer irregular terminal branches.

3. The reception container according to claim 2, **characterized in that** the transverse rods (6b) branching off the longitudinal rod (6a) are offset by 90 degrees relative to each other.

4. The reception container according to claim 2 or 3, **characterized in that** the longitudinal rod (6a) is provided at the two outer ends thereof with a crown of outwardly inclined upstanding fins (26a).

## Revendications

1. Dispositif d'irradiation d'échantillons de liquides organiques du corps, notamment de sang et/ou d'extraits tissulaires, par lumière UV, avec un appareil d'irradiation UV comprenant une lampe ultraviolette et avec un récipient qui est destiné à contenir les échantillons de liquides organiques du corps qu'il y a lieu d'irradier et est apte à être amené dans la zone de rayonnement de la lampe ultraviolette, un dispositif de chicanes (6, 26, 36), qui est destiné à la mise en turbulence d'un liquide organique du corps traversant le récipient (4) ou circulant à l'intérieur de celui-ci et qui comprend une pluralité de chicanes (6b, 36b, 36c) obligeant le liquide organique du corps à les contourner étant prévu, ce qui permet d'empêcher, dans une large mesure, un écoulement laminaire dans le liquide organique du corps,
**caractérisé en ce que**
le récipient est conformé en forme de tube et est adapté pour être placé dans une fixation pivotante de l'appareil d'irradiation UV
et que le dispositif de chicanes est réalisé sous forme d'un système de tiges (6) destiné à mettre en turbulence le liquide et comportant une tige longitudinale (6a) qui s'étend suivant la direction de l'axe du tube et dont partent des tiges transversales (6b).

2. Récipient (4) qui laisse passer les rayons UV et au sein duquel est prévu un dispositif de chicanes (6, 26, 36) qui est destiné à la mise en turbulence d'un liquide organique du corps traversant le récipient (4) ou circulant à l'intérieur de celui-ci et qui comprend une pluralité de chicanes (6b, 36b, 36c) obligeant le liquide organique du corps à les contourner, ce qui permet d'empêcher, dans une large mesure, un écoulement laminaire dans le liquide organique du corps,
**caractérisé en ce que**
le récipient est conformé en forme de tube, que le dispositif de chicanes s'étend sur la longueur du récipient et est réalisé de manière à être constitué d'une tige longitudinale (6a ; 36a) qui s'étend suivant la direction de l'axe du tube et dont partent des tiges transversales (6b) ou d'un tronc qui s'étend sensiblement suivant la direction de l'axe du tube et qui se divise en une pluralité de branches (36b) présentant des ramifications irrégulières de plus en plus fines.

3. Récipient selon la revendication 2, **caractérisé en ce que** les tiges transversales (6b) partant de la tige longitudinale (6a) sont disposées en décalage angulaire de 90° les unes par rapport aux autres.

4. Récipient selon la revendication 2 ou 3, **caractérisé en ce que** la tige longitudinale (6a) est munie à ses deux extrémités extérieures d'une couronne de lamelles (26a) dressées obliquement vers l'extérieur.
